# FASCICULE DE BREVET EUROPEEN

(11) **EP 1 716 238 B1**
(45) Date de publication et mention de la délivrance du brevet: **06.12.2017**
(21) Numéro de dépôt: 04816445.3
(22) Date de dépôt: 20.12.2004
(51) Int. Cl.: C12N 15/82

(54) **METHODE POUR MODIFIER L'EXPRESSION GENIQUE D' UN CHAMPIGNON PHYTOPATHOGENE**
VERFAHREN ZUR MODIFIKATION DER GENEXPRESSION EINES PHYTOPATHOGENEN PILZES
METHOD FOR MODIFYING GENE EXPRESSION OF A PHYTOPATHOGENIC FUNGUS

(30) Priorité: 23.12.2003 FR 0315228; 02.07.2004 FR 0407373
(43) Date de publication de la demande: 02.11.2006
(73) Titulaire: Bayer S.A.S., 69009 Lyon (FR)
(72) Inventeur: BALTZ, Rachel, 69009 Lyon (FR); DUMAIN, Raphael, 69009 Lyon (FR); PEYRARD, Stéphane, 69300 Caluire et Cuire (FR); FERULLO, Jean-Marc, 14169 Berlin (DE); BEFFA, Roland, 69230 St Genis Laval (FR)
(74) Mandataire: Guitton, Carole
(86) Numéro de dépôt international: PCT/FR2004/003312
(87) Numéro de publication internationale: WO 2005/071091

(56) Documents cités:
- WO-A-00/26346
- WO-A-01/96584
- WO-A-99/32619
- US-A1- 2003 150 017
- WATERHOUSE ET AL: "Virus resistance and gene silencing in plants can be induced by simultaneous expression of sense and antisense RNA" PROCEEDINGS OF THE NATIONAL ACADEMY OF SCIENCES OF USA, NATIONAL ACADEMY OF SCIENCE. WASHINGTON, US, vol. 95, novembre 1998 (1998-11), pages 13959-13964, XP002114472 ISSN: 0027-8424 cité dans la demande
- TANG GUILIANG ET AL: "A biochemical framework for RNA silencing in plants." GENES AND DEVELOPMENT, vol. 17, no. 1, 1 janvier 2003 (2003-01-01), pages 49-63, XP002295273 ISSN: 0890-9369
- KADOTANI NAOKI ET AL: "RNA silencing in the phytopathogenic fungus Magnaporthe oryzae." MOLECULAR PLANT-MICROBE INTERACTIONS, vol. 16, no. 9, septembre 2003 (2003-09), pages 769-776, XP009036113 ISSN: 0894-0282

## Description

La présente invention a pour objet un système de contrôle du développement des champignons lors d'une attaque phytopathogène. Le système selon l'invention consiste à faire exprimer à la plante un construit permettant d'inhiber l'expression d'un gène essentiel au développement ou à la pathogénicité du champignon. Ce système est communément appelé interférence ARN. Les plantes ainsi transformées constituent l'un des aspects de la présente invention.

Les maladies des plantes causent des pertes de rendements considérables, il en résulte des pertes économiques pour les agriculteurs, mais également les nombreux dégâts nutritionnels pour les populations locales vivant de leur agriculture. Economiquement et écologiquement, il est très intéressant d'avoir des plantes résistantes à leurs pathogènes et plus particulièrement à leurs champignons en l'absence de produits phytosanitaires. A ce jour, différentes stratégies ont pu être employées :
- Les méthodes de sélection traditionnelle ont été utilisées pour développer des plantes spécifiquement résistantes à certains pathogènes. Cependant, ces méthodes sont limitées aux espèces pouvant être croisées et l'introgression de caractères de résistance à des pathogènes constitue un travail long et laborieux.
- L'utilisation d'un ARN antisens permet de diminuer l'expression d'un gène cible endogène (EP 240 208)
- L'utilisation d'un gène sens permet de diminuer l'expression d'un gène cible endogène, cette technologie est appelée la cosuppression (EP 465 572).

La technologie utilisée dans le cadre de la présente invention est l'interférence ARN ou RNAi. Le RNAi a notamment prouvé son efficacité lors d'injection d'ARN double brins (dsRNA) dans le nématode *Caenorhabditis elegans* (Fire et al. 1998, Nature 391 : 806-811 et Montgomery et al., 1998, PNAS 95: 15502-15507, WO99/32619).

L'expression dans un organisme d'une séquence homologue au gène d'intérêt capable d'induire la formation d'ARN double brin de petite taille permet, de façon très spécifique, d'éteindre ce gène et d'observer le phénotype qui en résulte (Xiao et al., 2003, Plant Mol Biol., 52(5) : 957-66). L'exemple le plus marquant qui illustre cette facilité et celui d'insectes nourris avec des bactéries exprimant des petits ARN double brin correspondant à un gène exprimé chez les insectes qui est alors inhibé (WO 01/37654).

Le dsRNA déclenche la dégradation spécifique d'un ARN homologue uniquement dans la région d'identité avec le dsRNA (Zamore et al., 2000, Cell, 101 : 25-33, Tang et al., 2003 Gene Dev., 17(1) : 49-63). Le dsRNA est une molécule d'ARN qui contient une séquence double brin d'au moins 25 paires de bases (pb) incluant un brin sens et un brin antisens. Les molécules de dsRNA sont également caractérisées par le degré de complémentarité très important entre les deux brins d'ARN complémentaires. Le dsRNA est dégradé en fragments d'ARN de 19 à 25 nucléotides (siRNA) et les sites de clivages sur l'ARN cible sont régulièrement espacés de 19 à 25 nucléotides. Les petits siRNA en résultant présentent un degré d'identité très élevé par rapport à l'ARN cible, cependant des mismatches de 3 à 4 nucléotides entre le siRNA et la portion correspondant de l'ARN cible permet quand même au système de fonctionner (Tang et al., 2003, Genes Dev., 17 : 49-63). Il a donc été suggéré que ces fragments de 19 à 25 nucléotides constituent des guides ARN pour la reconnaissance de la cible (Zamore et al., 2000, Cell, 101 : 25-33). Ces petits ARN ont également été détectés dans des extraits préparés à partir de cellules Schneider 2 de *Drosophila melanogaster* qui avaient été transfectées avec des dsRNA avant la lyse des cellules (Hammond et al., 2000, Nature 404 : 293-296). Le rôle de guide des fragments de 19 à 25 nucléotides dans le clivage des ARNm est renforcé par l'observation selon laquelle ces fragments de 19 à 25 nucléotides isolés à partir de dsRNA sont capables d'intervenir dans la dégradation d'ARNm (Zamore et al., 2000, Cell, 101 : 25-33). Des molécules d'ARN de taille homologues s'accumulent également dans des tissus végétaux qui subissent le phénomène de PTGS (Post Transcriptional Gene Silencing, Hamilton et Baulcome, 1999, Science 286 : 950-952). Ces petits ARN peuvent réguler l'expression des gènes à trois niveaux différents :
- la transcription (TGS pour Transcriptional Gene Silencing),
- la dégradation des ARN messagers (PTGS pour Post Transcriptional Gene Silencing),
- la traduction.

La régulation faisant intervenir la dégradation des ARN messagers semblent exister chez tous les eucaryotes tandis que la régulation au niveau de la transcription n'a été décrite que chez les plantes, la drosophile et *C. elegans.* Quant à la régulation de la traduction elle a été caractérisée chez *C. elegans* et la drosophile et semble exister également chez les mammifères (Hannon, 2002, Nature, 418(6894) : 244-51). Dans la littérature, pour faire référence à ce phénomène, selon les organismes chez lesquels il est étudié, on parle de RNAi, de PTGS, de co-suppression ou de quelling (réservé aux champignons).

L'introduction de dsRNA a été réalisée chez les plantes pour induire une interférence (silencing) d'un gène cible endogène (Hamilton et al., 1998, Plant J, 15 : 737-746, WO99/15682), pour induire une résistance à des virus à ARN grâce à l'utilisation de transgène exprimant un dsRNA ayant une identité substantielle par rapport aux gènes viraux (Waterhouse et al., 1998, PNAS 95 : 13959-13964, Pandolfini et al., 2003, Biotechnol., 25; 3(1) : 7, WO98/36083, WO99/15682, US 5,175,102) mais également pour induire une résistance aux nématodes (Chuang et Meyerowitz, 2000, PNAS, 97 : 4985-4990, WO01/96584) ou encore à la bactérie *Agrobacterium* (WO00/26346, Escobar et al., 2001, Proc. Natl. Acad. Sci. U S A., 98(23):13437-13442).

Dans le cas de l'attaque d'une plante par une bactérie ou par un virus les mécanismes d'interaction entre la plante et le pathogène font clairement intervenir des transferts d'acides nucléiques. En effet, dans le cas d'*Agrobacterium tumefaciens,* les mécanismes de pathogénicité comportent deux étapes : la première correspond à un transfert de gène horizontal et à l'intégration de ce(s) gène(s) dans la plante (c'est la transformation), la deuxième correspond à des événements de post-intégration se produisant dans la plante (c'est la tumorigénèse; Escobar et al., 2001, Proc. Natl. Acad. Sci. U S A., 98(23):13437-42) basés sur l'utilisation par la plante du matériel génétique du pathogène. Dans le cas de l'infection du tabac par le *Plum Pox Virus* (PPV), c'est le transfert dans la plante de l'ARN simple brin du virus qui permet la synthèse des protéines de capside et des polymérases nécessaires à la propagation de l'infection (Pandolfini *et al.,* 2003). Il y a donc un lien et des échanges très élaborés au niveau génétique entre la plante et son pathogène, les siRNA sont transférés au cours de ses échanges. Les mécanismes d'infection d'une plante par un champignon phytopathogène quant à eux ne font pas intervenir de transfert de gène.

La présente invention a pour objet la création d'un construit et son utilisation dans des plantes ou des cellules génétiquement modifiées afin de les rendre résistantes à des champignons pathogènes. La technologie utilisée est basée sur le mécanisme d'interférence ARN. Ces plantes présentent l'avantage de ne pas produire de protéines, les risques de problèmes allergiques sont considérablement réduits : les seuls éléments surexprimés étant des ARN. De plus, le mécanisme d'ARN interférence est un système exponentiel et autoréplicatif, ce qui signifie qu'il suffit de l'induire pour qu'il se maintienne dans l'organisme.

Un des objets de la présente invention concerne un procédé de production d'une plante résistante à un champignon phytopathogène comprenant les étapes suivantes :
a) introduction dans une cellule végétale d'un construit comprenant :
   - une séquence de régulation promotrice fonctionnelle dans les cellules végétales,
   - une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
   - une séquence de régulation terminatrice,
b) mise en culture des cellules transformées dans des conditions permettant la transcription du construit
c) sélection des cellules transformées
d) régénération de plantes à partir des cellules transformées.

Un autre objet de la présente invention concerne un procédé de production d'une plante résistante à un champignon phytopathogène comprenant les étapes suivantes :
a) introduction dans une plante d'un construit comprenant :
   - une séquence de régulation promotrice fonctionnelle dans les cellules végétales,
   - une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
   - une séquence de régulation terminatrice,
b) mise en culture des plantes transformées dans des conditions permettant la transcription du construit
c) sélection des plantes transformées.

Un autre objet de la présente invention concerne un procédé de production d'une cellule végétale résistante à un champignon phytopathogène comprenant les étapes suivantes :
a) introduction dans une cellule végétale d'un construit comprenant :
   - une séquence de régulation promotrice fonctionnelle dans les cellules végétales,
   - une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
   - une séquence de régulation terminatrice,
b) sélection des cellules transformées,
c) mise en culture des cellules transformées dans des conditions permettant la transcription du construit.

L'invention concerne également une plante résistante à un champignon phytopathogène comprenant un construit caractérisé en ce qu'il comprend :
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice.

Les cellules végétales résistantes à un champignon phytopathogène comprenant un construit caractérisé en ce qu'il comprend
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice
sont un autre objet de la présente invention.

Un autre aspect de la présente invention concerne l'utilisation d'un construit pour créer une cellule végétale ou une plante résistante à un champignon, ledit construit comprenant, une séquence de régulation promotrice fonctionnelle dans les cellules végétales, une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel, une séquence de régulation terminatrice.

L'invention a également pour objet un procédé pour l'identification d'un gène essentiel au développement ou à la pathogénicité d'un champignon phytopathogène comprenant les étapes suivantes :
a) transformation d'une cellule végétale ou d'une plante avec un construit comprenant
   - une séquence de régulation promotrice fonctionnelle dans les cellules végétales,
   - une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
   - une séquence de régulation terminatrice.
b) mise en contact des cellules ou des plantes ainsi transformées avec le champignon phytopathogène
c) étude du phénotype résultant,
d) caractérisation du gène correspondant à la séquence de nucléotides ainsi insérée.

Un procédé pour inhiber l'expression d'un gène de champignon comprenant les étapes suivantes :
a) transformation d'une cellule végétale ou d'une plante avec un construit comprenant
   - une séquence de régulation promotrice fonctionnelle dans les cellules végétales,
   - une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
   - une séquence de régulation terminatrice.
b) Sélection des cellules transformées
c) mise en culture des cellules ainsi transformées dans des conditions permettant la transcription dudit construit
d) mise en contact des cellules avec le champignon.
constitue encore un autre aspect de l'invention

L'invention concerne encore un procédé pour réduire l'expression d'un gène de champignon comprenant les étapes suivantes :
a) transformation d'une cellule végétale ou d'une plante avec un construit comprenant
   - une séquence de régulation promotrice fonctionnelle dans les cellules végétales,
   - une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
   - une séquence de régulation terminatrice.
b) Sélection des cellules transformées
c) mise en culture des cellules ainsi transformées dans des conditions permettant la transcription dudit construit
d) mise en contact des cellules avec le champignon.

Par "séquence de régulation promotrice" : on entend selon l'invention toute séquence de régulation promotrice d'un gène s'exprimant naturellement dans les plantes en particulier un promoteur s'exprimant notamment dans les feuilles des plantes, comme par exemple des promoteurs dits constitutifs d'origine bactérienne, virale ou végétale ou encore des promoteurs dits lumière dépendants comme celui d'un gène de la petite sous-unité de ribulose-biscarboxylase/oxygénase (RuBisCO) de plante ou tout promoteur convenable connu pouvant être utilisé. Parmi les promoteurs d'origine végétale on citera les promoteurs d'histone tels que décrits dans la demande EP 0 507 698, ou le promoteur d'actine de riz (US 5,641,876). Parmi les promoteurs d'un gène de virus de plante, on citera celui de la mosaïque du choux fleur (CAMV 19S ou 35S), de la mosaïque du manioc (CsVMV : WO97/48819) ou le promoteur du circovirus (AU 689 311). On peut encore utiliser une séquence de régulation promotrice spécifique de régions ou de tissus particuliers des plantes, et plus particulièrement des promoteurs spécifiques des graines (Datla, R. et al., 1997, Biotechnology Ann. Rev., 3, 269-296), notamment les promoteurs de la napine (EP 255 378), de la phaséoline, de la gluténine, de l'héliantinine (WO 92/17580), de l'albumine (WO 98/45460), de l'oélosine (WO 98/45461). Un promoteur inductible peut être également employé, il peut être avantageusement choisi parmi les promoteurs de phénylalanine ammoniac lyase (PAL), d'HMG-CoA réductase (HMG), de chitinases, de glucanases, d'inhibiteurs de protéinase (PI), de gènes de la famille PR1, de la nopaline synthase (nos) ou du gène vspB (US 5 670 349), le promoteur HMG2 (US 5 670 349), le promoteur de la béta-galactosidase (ABG1) de pomme ou le promoteur de l'amino cyclopropane carboxylate synthase (ACC synthase) de pomme (WO 98/45445).

Par "séquence de régulation terminatrice", on entend toute séquence fonctionnelle dans les cellules végétales ou les plantes comprenant également les séquences de polyadénylation qu'elles soient d'origine bactérienne, comme par exemple le terminateur nos ou ocs d'*Agrobacterium tumefaciens*, d'origine virale, comme par exemple le terminateur du CaMV 35S, ou encore d'origine végétale, comme par exemple un terminateur d'histone tel que décrit dans la demande EP 0 633 317.

L'étape de sélection permettant d'identifier les cellules et/ou les plantes transformées ayant intégré le construit selon l'invention peut être réalisée grâce à la présence d'un gène de sélection présent dans le construit selon l'invention ou dans le plasmide utilisé pour la transformation des cellules ou des plantes et comprenant ledit construit. Le gène de sélection peut être sous la forme d'un gène chimère comprenant les éléments suivants, liés de manière opérationnelle dans le sens de la transcription : une séquence de régulation promotrice fonctionnelle dans les cellules végétales, une séquence codant pour un marqueur de sélection, une séquence de régulation terminatrice fonctionnelle dans les cellules végétales.

Parmi les marqueurs de sélection utilisables, on peut citer des marqueurs contenant des gènes de résistance aux antibiotiques tel que, par exemple, celui du gène de l'hygromycine phosphotransférase (Gritz et al., 1983, Gene 25:179-188), de la néomycine phospho transférase II induisant la résistance à la kanamycine (Wirtz et al., 1987, DNA, 6(3) : 245-253), de l'aminoglycoside 3"-adenyltransférase mais également des marqueurs contenant des gènes de tolérance aux herbicides tel que le gène *bar* (White et al., NAR 18:1062, 1990) pour la tolérance au bialaphos, le gène EPSPS (US 5,188,642) pour la tolérance au glyphosate ou encore le gène HPPD (WO 96/38567) pour la tolérance aux isoxazoles. On peut également citer des gènes codant pour des enzymes facilement identifiables comme l'enzyme GUS, des gènes codant pour des pigments ou des enzymes régulant la production de pigments dans les cellules transformées. De tels gènes marqueurs de sélection sont notamment décrits dans les demandes de brevet WO 91/02071, WO 95/06128, WO 96/38567, et WO 97/04103.

De manière préférentielle, la séquence nucléotidique du gène essentiel au champignon ou à sa pathogénicité (gène cible) correspond à une région qui est transcrite et plus particulièrement qui est transcrite et traduite.

La longueur de la séquence nucléotidique sens a une taille minimum de 19 nucléotides.

La séquence sens comprend une séquence essentiellement homologue à un gène essentiel au champignon où à sa pathogénicité. En effet et de manière préférentielle, la séquence de nucléotides sens et la séquence nucléotidique du gène cible de champignon présentent un degré d'identité d'au moins 50%, de préférence d'au moins 70%. De manière tout à fait préférentielle le degré d'identité est d'au moins 85%, et de manière tout à fait préférentielle, le degré d'identité est de 100%.

Cependant, il est nécessaire que la séquence nucléotidique sens comprenne toujours une séquence d'environ 19 nucléotides, particulièrement de 20 nucléotides et plus particulièrement de 25 nucléotides présentant au moins 80% d'identité avec la portion correspondante du gène cible et de manière tout à fait préférentielle 100% d'identité.

Dans l'un des aspects de l'invention, les séquences sens et antisens sont de tailles identiques. Selon un autre aspect de l'invention la séquence sens est de taille supérieure à la séquence antisens. A titre d'exemple, la taille de de la séquence sens peut présenter une taille superieure de l'ordre de 200 nucléotides par rapport à la séquence antisens. Dans un autre aspect de l'invention, la séquence antisens est de taille supérieure à la séquence sens.

La séquence antisens comprend une séquence essentiellement homologue à la séquence complémentaire du gène essentiel au champignon où à sa pathogénicité. En effet et de manière préférentielle, la séquence de nucléotides antisens et la séquence complémentaire du gène cible de champignon présentent un degré d'identité d'au moins 50%, de préférence d'au moins 70%. De manière tout à fait préférentielle le degré d'identité est d'au moins 85%, et de manière tout à fait préférentielle, le degré d'identité est de 100%.
Cependant, il est nécessaire que la séquence nucléotidique antisens comprenne toujours une séquence d'environ 19 nucléotides, particulièrement de 20 nucléotides et plus particulièrement de 25 nucléotides présentant au moins 80% d'identité avec la portion correspondante du gène cible et de manière tout à fait préférentielle 100% d'identité.

L'hybridation moléculaire est une réaction d'appariement qui s'effectue entre des brins complémentaires de polynucléotides présentant un certain degré d'identité entre leurs séquences nucléotidiques. Plus l'identité de séquence entre des polynucléotides est forte, plus l'hybridation entre lesdits polynucléotides est possible et aisée, et plus la probabilité que ces polynucléotides codent pour des protéines aux propriétés équivalentes est forte.

Le degré d'identité entre deux polynucléotides homologues est obtenu par comparaison de leurs séquences et est généralement exprimé par un pourcentage de nucléotides identiques entre ces séquences. Ce degré d'identité est mesuré sur une longueur de séquence donnée, la plus courte des séquences comparées déterminant la taille de séquence sur laquelle le degré d'identité des séquences homologues est mesuré. L'invention couvre donc des polynucléotides présentant une ou plusieurs modifications de séquence par rapport à l'autre et étant homologues du gène essentiel au champignon ou à sa pathogénicité.

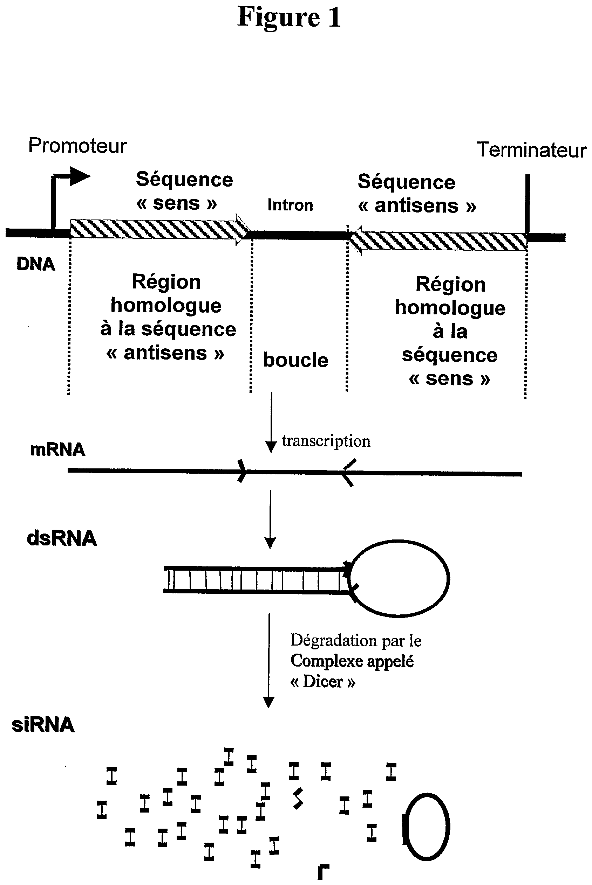

La séquence d'ADN selon l'invention peut revêtir deux aspects, dans le premier, elle comprend deux séquences de nucléotides sens et antisens séparées par un intron ne présentant pas d'homologie avec le gène du champignon. La figure 1 décrit une séquence de régulation promotrice fonctionnelle dans les cellules végétales se trouve devant la séquence de nucléotides en orientation sens du gène, suivie d'un intron et par la séquence de nucléotides en orientation antisens de ce même gène. Une séquence de régulation terminatrice se trouve à la fin du construit sens/intron/antisens. La séquence clonée en orientation sens et antisens est celle dont on veut inhiber l'expression dans le pathogène. La transcription de cette séquence d'ADN ("ADN" sur la figure) donne ainsi un grand ARN simple brin ("mRNA" sur la figure) correspondant au construit sens/intron/antisens. Ce long transcript d'ARN peut être détecté en RT-PCR. Les séquences sens et antisens étant homologues, elles vont s'apparier, l'intron qui les sépare joue le rôle de boucle permettant le repliement. Un dsRNA est alors obtenu ("dsRNA" sur la figure) sur l'ensemble des régions homologues. Le dsRNA est ensuite dégradé spécifiquement par un complexe enzymatique appelé "DICER". La dégradation des dsRNA forme alors des siRNA ("siRNA" sur la figure), petits ARN double brins ayant une taille comprise entre 19 et 25 bases. Ce sont alors les siRNA qui, en s'appariant aux ARN transcrits issus du gène cible entraîneront leur dégradation via la machinerie enzymatique de la plante.

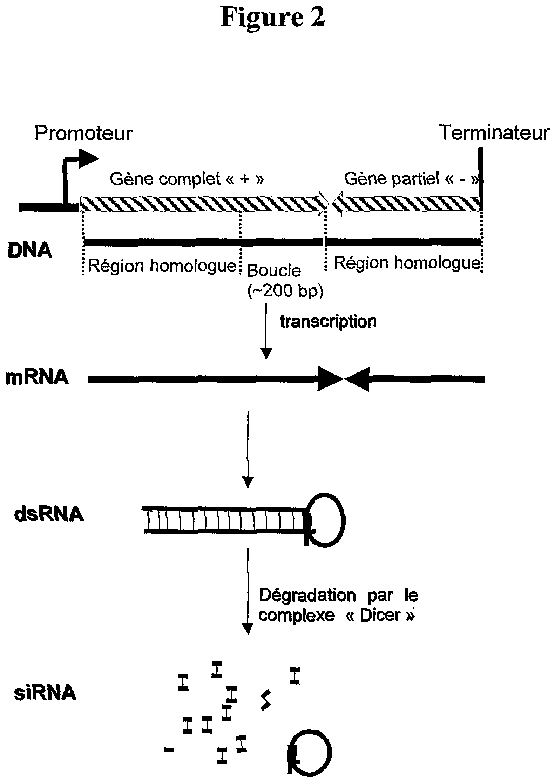

Dans le second aspect, la séquence d'ADN comprend deux séquences de nucléotides sens et antisens de tailles différentes, la structure en boucle correspondant à la partie de la séquence nucléotidique ne présentant pas d'homologie avec l'autre séquence nucléotidique. La figure 2 représente une séquence de régulation promotrice fonctionnelle dans les cellules végétales se trouve devant la séquence sens du gène, suivie par la séquence antisens partielle de ce même gène. Une séquence de régulation terminatrice se trouve à la fin du construit sens/antisens. La séquence de nucléotides clonée en orientation sens est essentiellement homologue à la séquence du gène cible dont on veut inhiber l'expression. La séquence nucléotidique antisens est essentiellement homologue au brin complémentaire de la séquence dudit gène cible. La transcription de cette séquence d'ADN ("ADN" sur la figure) donne ainsi un grand ARN simple brin ("mRNA" sur la figure) correspondant au construit sens/antisens (ce long transcript d'ARN peut être détecté en RT-PCR). Les séquences sens-antisens homologues s'apparient. Un dsRNA est alors obtenu ("dsRNA" sur la figure) sur l'ensemble des régions homologues. Le dsRNA est ensuite dégradé spécifiquement par un complexe enzymatique appelé "DICER". La dégradation des dsRNA forme alors des siRNA ("siRNA" sur la figure), petits ARN double brins ayant une taille comprise entre 19 et 25 bases. Ce sont alors les siRNA qui, en s'appariant aux ARN cibles, entraîneront leur dégradation via la machinerie enzymatique de la plante.

Les séquences nucléotidiques selon la présente invention peuvent être complémentaires d'un gène essentiel au champignon ou à sa pathogénicité.

Par "gène essentiel au champignon", on entend selon l'invention un gène dont l'inhibition par une molécule fongicide ou sa mutation entraîne la mort du champignon ou un arrêt de son développement. On peut citer à titre d'exemple les gènes suivants :
- gène codant pour la béta-tubuline (Katiyar et al., 1994, Antimicrob. Agents Chemother., 38(9) : 2086-90)
- de l'acétohydroxyacide isomérase (*ilv5*) intervenant dans la voie de biosynthèse des acides aminés à chaîne ramifiée (WO 03/022056),
- de la C14-deméthylase (*erg11*) ou de la C24-méthyltransférase (*erg6*) intervenant dans la voie de synthèse de l'ergostérol (Barrett et Dixon, 1995, Acta Biochem. Pol., 42(4) : 465-479)
- inositol phosphoceramide synthase (*aur1*) intervenant dans le transfert de l'inositol phosphocéramide sur la céramide dans la voie de biosynthèse des sphingolipides (Nagiec et al., 1997, J. Biol. Chem., 272(15) : 9809-9817) et de l'inositol phosphoryl transferase (*ipt1*).
- Glucane synthase et chitine synthase (Kang et al., 2001, Pest. Mana. Sci., 57(6) : 491-500, Binks et al., 1993, J. Gen. Microbiol., 139(6) : 1371-1377.
- des facteurs ribosomiques (*ef2* et *ef3*, Belfiel et Tuite, 1993, Mol. Microbiol., 9(3) : 411-418).
- *met4* et *met30* (Aoki et al., 1996, Antimicrob. Agents Chemother, 40(1) : 127-132.

Dans un mode de réalisation de l'invention les plantes ou des cellules végétales comprennent une séquence d'ADN complémentaire d'un gène essentiel au champignon, ledit gène essentiel étant représenté par l'identifiant de séquence SEQ ID No 4.

L'invention concerne également une méthode de production d'une cellule de tabac résistante au champignon *Cercospora nicotianae* par introduction d'un construit comprenant une séquence ADN représentée par l'identifiant de séquence SEQ ID No 4.

Par "gène essentiel à la pathogénicité du champignon", on entend selon l'invention un gène dont l'inhibition n'est pas létale pour le champignon mais inhibe son pouvoir pathogène. A titre d'exemple, on peut citer les gènes suivants :
- *763* de *Magnaporthe grisea* (WO 01 /75115).
- gène codant pour la polygalacturonase (Bonnin et al., 2001, Biochem. Biophys. Acta, 1526(3) : 301-309.
- *tri5* de *Fusarium graminearum,* intervenant dans la voie de biosynthèse des trichothécènes (Kimura et al., 2003, FEBS, 539(1-3) : 105-110.
- *fum5* de *Fusarium monoliforme,* intervenant dans la voie de biosynthèse des toxines de ce champignon (Proctor et al., 1999, Fungal Genet. Biol., 27(1) :100-112, Proctor et al., 2003, Fungal Genet. Biol., 38(2) : 237-249).
- *but* de *Magnoporthe grisea* intervenant dans la voie de biosynthèse de la mélanine (Kawamura et al., 1997, Mol. Plant Microbe Interact, 10(4) : 446-453.

Dans un autre mode de réalisation de l'invention les plantes ou des cellules végétales comprennent une séquence d'ADN complémentaire d'un gène essentiel à la pathogénicité du champignon, ledit gène étant représenté par l'identifiant de séquence SEQ ID No 13

L'invention concerne également une méthode de production d'une cellule de riz résistante au champignon *Magnoporthe grisea* par introduction d'un construit comprenant une séquence ADN représentée par l'identifiant de séquence SEQ ID No 13.

Les plantes et les cellules transformées selon l'invention peuvent être des monocotylédones ou des dicotylédones. De préférence, ces plantes sont des plantes d'intérêt agronomique.

Les cellules végétales et les plantes de la présente invention peuvent être des monocotylédones comme le blé, le maïs ou le riz.

Un mode de réalisation particulier de la présente invention correspond à une plante de riz résistante à *Magnaporthe grisea* comprenant une séquence ADN représentée par l'identifiant de séquence SEQ ID No 13.

Les cellules végétales et les plantes de la présente invention peuvent être des dicotylédones comme le colza, le soja, le coton ou le tabac.

Un mode de réalisation particulier de la présente invention correspond à une plante de tabac résistante à *Cercospora nicotianae* comprenant une séquence ADN représentée par l'identifiant de séquence SEQ ID No 4.

Ladite plante peut être obtenue par régénération des cellules transformées selon l'invention.

Pour obtenir les cellules ou des plantes selon l'invention, l'homme du métier peut utiliser une des nombreuses méthodes de transformation connues.

Une de ces méthodes consiste à mettre les cellules ou tissus des organismes hôtes à transformer en présence de polyéthylène glycol (PEG) et des vecteurs de l'invention (Chang et Cohen, 1979, Mol. Gen. Genet. 168(1), 111-115; Mercenier et Chassy, 1988, Biochimie 70(4), 503-517). L'électroporation est une autre méthode qui consiste à soumettre les cellules ou tissus à transformer et les vecteurs de l'invention à un champ électrique (Andreason et Evans, 1988, Biotechniques 6(7), 650-660; Shigekawa and Dower, 1989, Aust. J. Biotechnol. 3(1), 56-62). Une autre méthode consiste à directement injecter les vecteurs dans les cellules ou les tissus par micro-injection (Gordon et Ruddle, 1985, Gene 33(2), 121-136). De manière avantageuse, la méthode dite de "biolistique" pourra être utilisée. Elle consiste à bombarder des cellules ou des tissus avec des particules sur lesquelles sont adsorbés les vecteurs de l'invention (Bruce et al., 1989, Proc. Natl. Acad. Sci. USA 86(24), 9692-9696; Klein et al., 1992, Biotechnology 10(3), 286-291; US Patent No. 4,945,050). De manière préférentielle, la transformation de cellules ou tissus végétaux peut se faire à l'aide de bactéries du genre *Agrobacterium,* de préférence par infection des cellules ou tissus desdites plantes par *A*. *tumefaciens* (Knopf, 1979, Subcell. Biochem. 6, 143-173; Shaw et al., 1983, Gene 23(3):315-330) ou *A*. *rhizogenes* (Bevan et Chilton, 1982, Annu. Rev. Genet. 16:357-384; Tepfer et Casse-Delbart, 1987, Microbiol. Sci. 4(1), 24-28). De manière préférentielle, la transformation de cellules ou tissus végétaux par *Agrobacterium tumefaciens* est réalisée selon le protocole décrit par Hiei et al. (1994, Plant J. 6(2) : 271-282). L'homme du métier fera le choix de la méthode appropriée en fonction de la nature des organismes hôtes à transformer.

Les plantes selon l'invention contiennent des cellules végétales transformées telles que définies ci-dessus. En particulier, les plantes transformées sont susceptibles d'être obtenues par régénération des cellules végétales transformées décrites ci-dessus. La régénération est obtenue par tout procédé approprié qui dépend de la nature de l'espèce.

L'invention comprend également des parties de ces plantes, et la descendance de ces plantes. On entend par "partie de ces plantes", tout organe de ces plantes, qu'il soit aérien ou souterrain. Les organes aériens sont les tiges, les feuilles, et les fleurs comprenant les organes reproducteurs mâles et femelles. Les organes souterrains sont principalement les racines, mais ils peuvent également être des tubercules. Par "descendance", on entend principalement les graines contenant les embryons issus de la reproduction de ces plantes entre-elles. Par extension, le terme "descendance" s'applique à toutes les graines formées à chaque nouvelle génération issue de croisements entre les plantes transformées selon l'invention. Une descendance et des graines peuvent également être obtenus par multiplication végétative desdites plantes transformées. Les graines selon l'invention peuvent être enrobées d'une composition agrochimique comprenant au moins un produit actif possédant une activité sélectionnée parmi les activités fongicide, herbicide, insecticide, nématicide, bactéricide ou virucide.

Les différents aspects de l'invention seront mieux compris à l'aide des exemples expérimentaux ci-dessous.

Toutes les méthodes ou opérations décrites ci-dessous sont données à titre d'exemple et correspondent à un choix, effectué parmi les différentes méthodes disponibles pour parvenir au même résultat. Ce choix n'a aucune incidence sur la qualité du résultat et par conséquent, toute méthode adaptée peut être utilisée par l'homme de l'art pour parvenir au même résultat. En particulier, et à moins qu'il n'en soit précisé autrement dans les exemples, toutes les techniques d'ADN recombinant employées sont mises en oeuvre selon les protocoles standards décrits dans Sambrook and Russel (2001, Molecular cloning: A laboratory manual, Third edition, Cold Spring Harbor Laboratory Press, NY), in Ausubel et al. (1994, Current Protocols in Molecular Biology, Current protocols, USA, Volumes 1 and 2), and in Brown (1998, Molecular Biology LabFax, Second edition, Academic Press, UK). Des matériels et méthodes standards pour la biologie moléculaire des plantes sont décrits dans Croy R.D.D. (1993, Plant Molecular Biology LabFax, BIOS Scientific Publications Ltd (UK) and Blackwell Scientific Publications (UK). Des matériels et méthodes standards pour la PCR (Polymerase Chain Reaction) sont également décrits dans Dieffenbach and Dveksler (1995, PCR Primer: A laboratory manual, Cold Spring Harbor Laboratory Press, NY) et dans McPherson et al. (2000, PCR - Basics: From background to bench, First edition, Springer Verlag, Germany).

### Exemple 1 : Création d'un tabac résistant à Cercospora nicotianae

*Cercospora nicotianae* est un pathogène du tabac (*Nicotiana tabacum*). Le gène cible choisi chez *Cercospora nicotianae* est le gène codant pour la béta-tubuline. Les tubules sont des structures dynamiques présentes dans tous les types cellulaires. Dans les cellules qui ont la capacité de se diviser, les microtubules sont à la base de la formation du fuseau mitotique, pour les autres types cellulaires, ils constituent un réseau cytoplasmique essentiel pour l'organisation du noyau et des organelles dans l'espace cytoplasmique. Les microtubules sont des hétérodimères d'alpha- et béta-tubuline. La béta-tubuline est une protéine vitale pour le pathogène et est notamment la cible des benzimidazoles (Katiyar et al., 1994, Antimicrob. Agents Chemother, 38(9) : 2086-2090).

### 1. - construit utilisé pour transformer des cellules de tabac

Le construit utilisé pour transformer le tabac est le plasmide nommé pPAF 115, il comprend les marqueurs de sélection suivants : un gène de résistance à la kanamycine sous le contrôle du promoteur nos, un gène de résistance à la spectinomycine (*aadA*) sous le contrôle d'un promoteur bactérien. Le promoteur CaMV 35S permet la transcription de la séquence ADN comprenant une partie de la séquence sens de la béta-tubuline de *Cercospora nicotianae* représentée par l'identifiant de séquence SEQ ID No 1, elle-même suivie d'un intron représenté par l'identifiant de séquence SEQ ID No 2 et de la séquence antisens de cette même béta-tubuline représentée par l'identifiant de séquence SEQ ID No 3. Un terminateur ocs est en aval de l'ensemble sens-intron-antisens. Cet ensemble est représenté par l'identifiant de séquence SEQ ID No 4.
La variété de *Nicotiana tabacum* Petit Havana est transformée avec ce vecteur pPAF 115.

### 2. - Transformation de tissus foliaires de tabac par la solution d'Agrobacterium transformé avec le plasmide pPAF115.

La solution d'*Agrobacterium* (DO = 1) est lavée dans du MgSO4 10 mM. Des disques sont découpés dans des feuilles de tabac sauvage et sont désinfectés 10 min dans de l'éthanol à 75% puis sont incubés 5 min dans la solution d'*Agrobacterium.* Ils sont ensuite épongés, et déposés face inférieure contre une boite de Pétri contenant de la gélose MS 0.05-2 (Murashige & Skoog M5519, 30g/L saccharose, 0.05 ppm ANA, 2 ppm BAP, 7 g/L phytagar, pH = 5.7) pour une culture de trois jours. Les disques foliaires sont transférés sur des boîtes MS 0.05-2 en présence de spectinomycine et kanamycine durant deux à quatre semaines. Les disques foliaires sont ensuite placés sur du milieu MS 0-0 (Murashige & Skoog M5519, 30g/L saccharose, 7 g/L phytagar, pH = 5.7) contenant également les antibiotiques durant sept à dix jours. Les pousses de tabac issues des disques sont isolées et enracinées sur des boîtes gélosées MS 1/2-1/2 (½ Murashige & Skoog, 15 g/L saccharose, 7 g/L phytagar, pH = 5.7) contenant les antibiotiques. Une fois les deux premières feuilles apparues, les pousses sont placées dans de la terre pour que les plantes se développent.

### 3. - Analyses moléculaires effectuées sur le tabac transgénique

Trois événements différents ont été obtenus.

### 3.1. - PCR

Des premières expériences de PCR sont effectuées sur l'ADN génomique des différents événements afin de savoir si ceux-ci contiennent le construit selon l'invention. Des amorces permettant de détecter le gène de la kanamycine ainsi que la tubuline spécifique de *Cercospora* ont été utilisées.

| Evénement | **14** | **15** | **19** | **WT** |
|---|---|---|---|---|
| Kanamycine | + | + | + | - |
| Tubuline | + | + | + | - |

WT : wild type, plante non transformée.
Les séquences des amorces utilisées sont les suivantes:
Kanamycine :
- direct : 5'- CAA GAC CGA CCT GTC C- 3' (SEQ ID No 5)
- inverse : 5' - CCA TCC GAG TAC GTG C - 3' (SEQ ID No 6) Tubuline de *Cercospora nicotianae*:
- direct : 5' -ATC GAT AAC GAG GCC C- 3' (SEQ ID No 7)
- inverse : 5' -ACA TCG TAA GTC CTC GG- 3' (SEQ ID No 8)
L'ensemble des plantes contiennent le plasmide pPAF115 selon l'invention.

### 3.2. - RT-PCR quantitative

L'ARN des événements obtenus est extrait et une reverse transcription est réalisée avant d'effectuer une PCR quantitative. La reverse transcription se fait à partir de 4 µg d'ARN à partir d'amorces aléatoires. La PCR quantitative se fait selon ce schéma :

| | |
|---|---|
| ADNc 100 ng tubuline tabac | ADNc 100 ng tubuline *Cercospora* |
| ADNc 10 ng tubuline tabac | ADNc 10 ng tubuline *Cercospora* |
| ARN 100 ng tubuline tabac | ARN 100 ng tubuline *Cercospora* |
| ARN 10 ng tubuline tabac | ARN 10 ng tubuline *Cercospora* |

L'ARN est utilisé pour faire la réaction de PCR afin de s'assurer que la détection d'ADN obtenue avec l'ADNc est bien due à une amplification d'ADN complémentaire et non d'ADN génomique contaminant (l'amplification à partir de l'ARN doit être nulle).

La détection de l'ADN complémentaire de la tubuline de tabac grâce à l'utilisation des primers cités ci-dessous (SEQ ID No 9 et SEQ ID No 10) sert de niveau d'expression référence. C'est par rapport à cette expression de la tubuline de tabac qu'a été calculé le niveau d'expression du transgène.
Tubuline de tabac
- direct 5' - GAA AAC ACG TCC CTC G - 3' (SEQ ID No 9)
- inverse 5' - TCT TGC CGT AGT CCA C - 3' (SEQ ID No 10)
Pour toutes les expériences :
- Les contrôles ARN sont négatifs, il n'y a donc pas de contamination d'ADN génomique.
- La différence d'expression entre les concentrations de 100 et 10 ng est constante et conforme aux attentes pour des dilutions 10 fois.

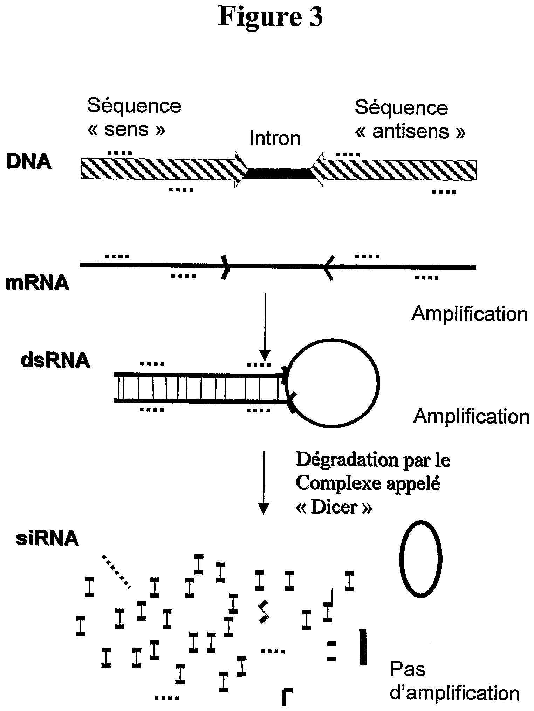

La figure 3 montre l'emplacement des primers de tubuline de *Cercospora nicotianae* sur les séquences d'ADN et d'ARN comprenant l'ensemble sens-intron-antisens tel que défini par l'identifiant de séquence SEQ ID No 4. Les pointillés représentent schématiquement les amorces avec lesquelles on fait la PCR quantitative (qPCR) après la réverse transcription (RT). La qPCR ne peut détecter les transcrits issus du construit selon l'invention que s'ils ne sont pas dégradés. La RT-qPCR ne détecte que les longs transcrits, mRNA ou dsRNA.

| **Evénement** | **Niveau de détection (RT-PCR quantitative)** | **Résultats** |
|---|---|---|
| WT | --- | • Pas de détection par PCR du construit |
| | | • Aucune détection d'ARN du construit |
| 14, 15 | +/- | • Détection positive mais faible par PCR du construit |
| | | • Détection faible d'ARN du construit |
| 19 | + | • Détection positive par PCR du construit |
| | | • Détection nette d'ARN du construit |

| | | |
|---|---|---|
| « + », signifie que le signal est nettement détecté par RT-qPCR « +/- », signifie que le signal est faiblement détecté «---» correspond à l'absence du transgène | | |

### 3.3. - RT-PCR quantitative en vue des tests in vivo

Les tests *in vivo* sont faits sur les plantes T0 décrites ci-dessus. Il s'agit des événements : 14, 15, 19 et WT. Pour ces 4 événements, des clones issus de la plante T0 initiale vont donc être utilisés pour effectuer les tests. Ces clones sont réalisés par découpage des feuilles de chaque événement cultivé à nouveau *in vitro* et régénéré (voir ci-dessus). Afin d'effectuer un contrôle rapide, une analyse en RT-PCR quantitative est effectuée sur un clone des événements 14, 19 et WT (14A, 19A et WT respectivement).

Tous les contrôles (ARN, WT, et référence interne (tubuline du tabac)) sont corrects. Suite à la validation des contrôles, on peut donner un niveau d'expression pour chaque clone : les résultats sont exprimés qualitativement en fonction des valeurs obtenues grâce à la PCR quantitative.

| **Evénement** | **Niveau d'expression (RT-PCR quantitative)** | **Résultats** |
|---|---|---|
| WT | --- | • Aucune détection par PCR du construit |
| | | • Aucune détection d'ARN du construit |
| 14A | +/- | • Détection positive mais faible par PCR du construit |
| | | • Détection faible d'ARN du construit |
| | | • Détection positive par PCR du construit |
| 19 A | + | • Détection nette d'ARN du construit |

| | | |
|---|---|---|
| « + », signifie que le signal est nettement détecté par RT-qPCR « +/- », signifie que le signal est faiblement détecté «---» correspond à l'absence du transgène | | |

### 4. - Tests pathologiques in vivo pour l'évaluation de la résistance des tabacs transgéniques à l'infection par Cercospora nicotianae.

### Matériel végétal :

Clones (au moins neuf) issus des événements T0 à un stade de développement des plantes comprenant au moins 5 feuilles développées.

Production : Serre S2 (25°C de jour / 20°C de nuit ; 60% HR ; Photopériode 15H de lumière)

### Inoculum :

Suspension de *Cercospora nicotianae* (environ 10⁴ spores/ml)

### Evaluation des symptômes :

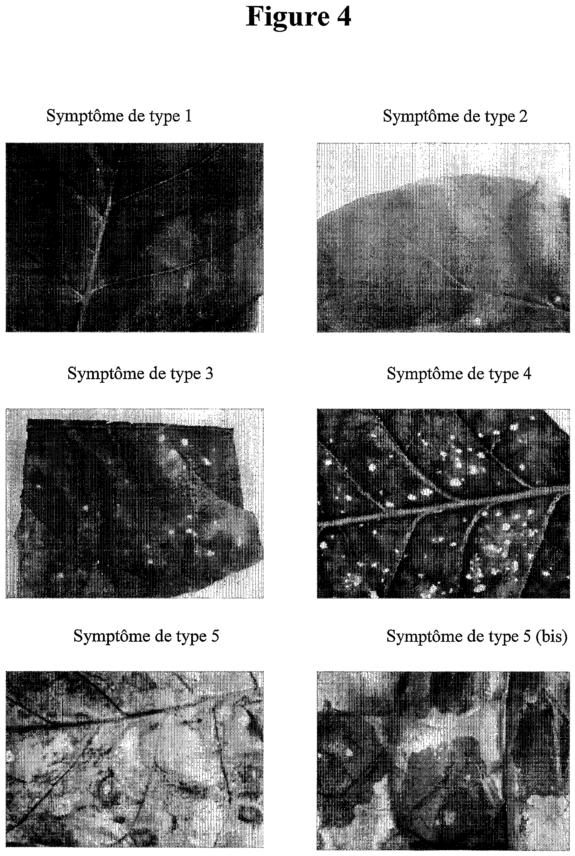

La détermination de l'intensité des symptômes se fait selon des critères biologiques en fonction de la taille et de l'allure des lésions constatées sur les feuilles. Ainsi comme on peut le voir sur la figure 4, la note 1 correspond à la visualisation de taches très légères sur la feuille, à peine perceptibles. La note 2 correspond à des taches nettes sur la feuille, mais très peu nombreuses. La note 3 correspond à des taches nettes et nombreuses sur la feuille. La note 4 correspond à des taches très nettes et très nombreuses avec présence de taches de grande taille. La note 5 correspond soit à une pourrissement total de la feuille, soit à de nombreuses tâches de très grosse taille.
Les différences entre 1, 2, 3, 4 et 5 correspondent donc à de réelles et nettes différences de symptômes. Ainsi, des plantes ayant des notes de symptômes de 4 ou 5 sont considérées comme très sévèrement touchées. Des plantes de note 3 sont touchées mais restent acceptables. Des plantes de note 2 sont faiblement touchées et celles de note 1 ne le sont que très faiblement.
Il y a par ailleurs trois lectures différentes :
1^{er} lecture : 10 jours après inoculation
2^{e} lecture : 21 jours après inoculation
3^{e} lecture : 1 mois après inoculation

D'autre part, la détermination du pourcentage de contamination de la surface foliaire varie de 0 % à 100 %. 0% correspond à une observation où aucun symptôme n'est visible sur la feuille, il semble y avoir une absence totale du pathogène. 100 % correspond à une feuille recouverte complètement par le pathogène, c'est à dire une feuille brune et pourrie. Le résultat de la lecture étant obtenue avec la moyenne des feuilles d'une même plante, on ne peut obtenir de valeurs si extrêmes. On peut ainsi considérer qu'au-delà de 30 % de surface foliaire contaminée, la plante est très sévèrement touchée par la maladie. En dessous de 20 %, la contamination peut être jugée faible et en dessous de 10-15 % comme très faible.

Les résultats sont présentés sous forme d'histogrammes correspondant aux moyennes des plantes par événement (Résultats à 28 jours après l'infection). L'intervalle de confiance est représenté pour chaque histogramme.
Pour chaque événement le nombre de plantes analysées est indiqué ci-dessous ainsi que le nombre de feuilles correspondant :
- WT : 19 plantes (87 feuilles)
- Evénement 14 : 14 plantes (67 feuilles)
- Evénement 15 : 21 plantes (102 feuilles)
- Evénement 19 : 12 plantes (60 feuilles)

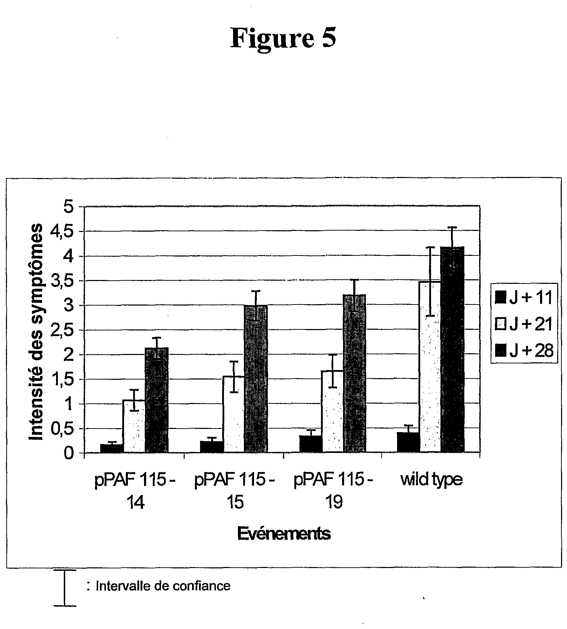

La figure 5 représente l'intensité des symptômes notée pour chaque événement.

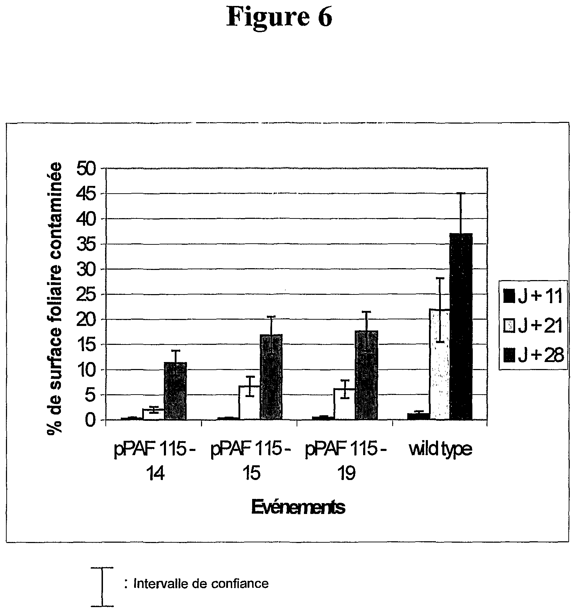

La figure 6 représente le pourcentage de surface foliaire contaminé pour chaque événement.

Les résultats du test *in vivo* sont clairement corrélés avec les analyses moléculaires : la détection des longs transcrits issus du construit selon l'invention est complètement corrélée avec une intensité des symptômes plus faible et donc une résistance de la plante transgénique.

| Evénement | Présence du transgène | RT-PCRq : | Tests pathologiques sur les tabacs T0 Résultats à 28 jours après infection | |
|---|---|---|---|---|
| | | Détection des longs ARN | % de surface foliaire contaminée (intervalle de confiance) | Intensité des symptômes (intervalle de confiance) |
| WT | Non | --- | 37% (6,6) | 4 (0,26) |
| 14 | Oui | -/+ | 11% (3,8) | 2 (0,37) |
| 15 | Oui | -/+ | 17% (3,85) | 3 (0,22) |
| 19 | Oui | + | 19 % (4,8) | 3,2 (0,34) |

| | | | | |
|---|---|---|---|---|
| -/+ : détection faible + : détection nette --- : pas de détection | | | | |

Les résultats présentés ci dessous sont établis à partir de tests validés statistiquement pour des essais biologiques. La population de chaque événement permet d'établir des intervalles de confiance robustes statistiquement.

### Exemple 2 : Création d'un riz résistant à Magnaporthe grisea

*Magnaporthe grisea* est un pathogène du riz (*Oryza sativa*). La cible choisie chez *Magnaporthe grisea* est le gène *buf.* Le gène *buf* est un gène essentiel à la pathogénicité de *Magnaporthe grisea.* En cas de délétion, *Magnaporthe grisea* est non-virulent et ne peut contaminer le riz. (Kawamura et al., 1997, Mol. Plant Microb. Interact., 10(4) : 446-53).

### 1. - Construit utilisé pour transformer des cellules de riz

Le construit utilisé pour transformer le riz est le plasmide nommé pPAF 74, il comprend le marqueur de sélection suivant : un gène de résistance à la gentamycine sous le contrôle d'un promoteur bactérien. Le promoteur CaMV 35S permet la transcription de la séquence ADN comprenant une partie de la séquence sens du gène *buf* représentée par l'identifiant de séquence SEQ ID No 11, elle-même suivie de la séquence antisens de ce même gène *buf* partiellement délété par rapport à la séquence sens représentée par l'identifiant de séquence SEQ ID No 12. Un terminateur Nos est en aval de l'ensemble sens-antisens lequel est représenté par l'identifiant de séquence SEQ ID No 13. La variété de *Oryza sativa* Nippon Bare est transformée avec ce vecteur pPAF 74.

### 2. - Transformation de tissus de riz par la solution d'Agrobacterium transformé avec le plasmide pPAF 74

Les embryons sont sortis du péricarpe et sont plongés dans une solution d'éthanol à 70% durant 1 min. Ils sont ensuite placés dans de l'eau de javel du commerce diluée au tiers pendant 30 min et agités de temps en temps. Ils sont enfin rincés 3 fois avec de l'eau stérile.

Les embryons ainsi décontaminés sont placés sur le flanc (12 par boite), sur milieu NB (Macro éléments N6, FeEDTA, Micro-éléments B5, Vitamines B5, Myo-inositol, Proline Glutamine, Hydrolysat de caséine, Saccharose, 2,4-D, Phytagel). La boite est laissée entrouverte sous la hotte pour bien sécher le grain (étuve à 28°C - obscurité - 17 à 20 jours). Après une période de 17 à 20 jours (28°C - obscurité), les petites unités embryogènes (nodules de diamètre de 0.5 à 1 mm) qui se forment à partir du cal primaire, au contact du milieu sont excisées, et repiquées sur milieu NB en boîte 100x15 (étuve à 28°C - obscurité - 10 jours).

Une culture d'Agrobactéries (3-5.10⁹ bactéries/ml (DO₆₀₀=1)) est utilisée pour la transformation.

Il faut ensuite inoculer et faire une coculture :
Des cals de 3 à 5 mm, bien ronds, rugueux, blanchâtres et opaques sont immergés dans la solution d'agrobactéries contenant le plasmide pPAF 74 (3-5.10⁹ bactéries/ml) (20ml par boite) durant 15 min sous une légère agitation.

Les cals sont transférés sur papier Whatman stérile et sont séchés. Les cals sont ensuite mis en culture sur milieu R2-CS (Macro éléments R2-I, Macro éléments R2-II, FeEDTA, Micro-éléments R2, Vitamines R2, Glucose, 2,4-D, Agarose de type I) dans l'obscurité, à une température de 25°C pendant 3 jours (10 cals par boite).

Les cals sont transférés sur milieu de sélection R2S (Macro éléments R2-I, Macro éléments R2-II, FeEDTA, Micro-éléments R2, Vitamines R2, Saccharose, 2,4-D, Agarose de type I, Vancomycine, Cefotaxime, Hygromycine) (28°C - obscurité - 2 semaines). Les cals sont repiqués sur milieu NBS (Macro éléments N6, FeEDTA, Micro-éléments B5, Vitamines B5, Myo-inositol, Proline, Glutamine, Hydrolysat de caséine, Saccharose, 2,4-D, Agarose de type I, Cefotaxime, Vancomycine, Hygromycine) (28°C - obscurité - 1 semaine). Etaler les proliférations (globules) autour de leur cal d'origine, pour les mettre en contact avec le milieu de sélection. (28°C - obscurité - 1 à 2 semaines)

Après il faut que les transformants entrent en maturation :
Les proliférations résistantes sont repiquées (globules bien développés, de couleur jaune-blanc) sur milieu PR-AG (Macro éléments N6 FeEDTA Micro-éléments B5 Vitamines B5 Myo-inositol Proline Glutamine Hydrolysat de caséine Saccharose ABA BAP ANA Agarose de type 1 Cefotaxime Hygromycine Vancomycine), en identifiant le cal d'origine (10 cals par boite). (28°C - obscurité - 8 jours)

Régénération des transformants :
Les cals sont repiqués sur milieu de régénération RN (Macro éléments N6, FeEDTA, Micro-éléments B5, Vitamines B5, Myo-inositol, Proline, Glutamine, Hydrolysat de caséine, Saccharose, BAP, ANA, Phytagel) (28°C - lumière continue- 3 semaines).

Les plantes régénérées sont repiquées en tube (Milieu MS, Saccharose, Phytagel).

Elles sont ensuite repiquées dans du terreau, acclimatées et cultivées en serre.

Acclimatation des plantes transformées :
Les plantules sont acclimatées lorsqu'elles se sont bien développées en tube (après environ 3-4 semaines). Sortir la plantule du tube. Passer les racines sous un filet d'eau pour éliminer la gélose. Couper l'extrémité des feuilles et des racines. Eliminer les feuilles mortes. Placer la plantule en pot, dans une petite serre ("petite maison verte") contenant 1-2 cm d'eau ou pas du tout pour éviter le pourrissement, porte fermée. Protéger le dessus de la petite serre avec du papier absorbant. Placer le tout en chambre de culture *in vitro.* Entrouvrir la porte graduellement, et éliminer le papier. Lorsque les racines sortent du pot (au bout d'environ 15 jours), repiquer les plantes dans du terreau, en pot.

Huit événements différents ont été obtenus.

### 3. - Analyses moléculaires effectuées sur le riz transgénique

### 3.1.-PCR

Des premières expériences de PCR sont effectuées sur l'ADN génomique des différents événements afin de savoir si ceux-ci contiennent le construit selon l'invention. Des amorces permettant de détecter le gène buf de *Magnaporthe* ont été utilisées.

| Evénement | **M** | **L** | **F** | **WT** |
|---|---|---|---|---|
| *buf* | + | + | + | - |

WT : wild type, plante non transformée.
Les séquences des amorces utilisées sont les suivantes :

### buf de Magnaporthe :

Direct : 5' - TGA CCG TGT CTT TAC CA -3' (SEQ ID No 16)
Indirect : 5' - AGC AAC CAC ATT AAC AGT -3' (SEQ ID No 17)

Toutes les plantes contiennent le plasmide selon l'invention.

### 3.2. RT-PCR quantitative :

L'ARN de ces 4 événements est extrait et une reverse transcription est réalisée avant d'effectuer une PCR quantitative. La reverse transcription se fait à partir de 4 µg d'ARN à partir d'amorces aléatoires. La PCR quantitative se fait selon ce schéma :

| | |
|---|---|
| ADNc 100 ng tubuline riz | ADNc 100 ng buf de Magnaporthe |
| ADNc 10 ng tubuline riz | ADNc 10 ng buf de Magnaporthe |
| ARN 100 ng tubuline riz | ARN 100 ng buf de Magnaporthe |
| ARN 10 ng tubuline riz | ARN 10 ng buf de Magnaporthe |

L'ARN est utilisé pour faire la réaction de PCR afin de s'assurer que la détection d'ADN obtenue avec l'ADN est bien due à une amplification d'ADN complémentaire et non d'ADN génomique contaminant (l'amplification à partir de l'ARN doit être nulle).

La détection de l'ADN complémentaire de la tubuline de tabac sert de niveau d'expression référence. C'est par rapport à cette expression de la tubuline de tabac qu'a été calculé le niveau d'expression du transgène.

Les séquences des amorces sont les suivantes :
Tubuline de riz :
Direct : 5' - CAT TGA CTT CAC GCG G -3' (SEQ ID No 14)
Indirect : 5' - GAC ACT GGA TTT GAC GTT -3' (SEQ ID No 15)

*buf* de Magnaporthe :
Direct : 5' - TGA CCG TGT CTT TAC CA -3' (SEQ ID No 16)
Indirect : 5' - AGC AAC CAC ATT AAC AGT -3' (SEQ ID No 17)

Pour toutes les expériences :
- Les contrôles ARN sont négatifs, il n'y a donc pas de contamination d'ADN génomique.
- La différence d'expression entre les concentrations de 100 et 10 ng est constante et conforme aux attentes pour des dilutions 10 fois.

| **Evénement** | **Niveau d'expression (RT-PCR quantitative)** | **Résultats** |
|---|---|---|
| WT | --- | • Aucune détection par PCR du construit |
| | | • Aucune détection d'ARN correspondant au gène *buf* |
| M | +/- | • Détection par PCR du construit |
| | | • Détection faible d'ARN correspondant au construit *buf* |
| L | + | • Détection par PCR du construit |
| | | • Détection nette d'ARN correspondant au construit *buf* |
| F | ++ | • Détection par PCR du construit |
| | | • Détection très nette d'ARN correspondant au construit *buf* |

| | | |
|---|---|---|
| « + », signifie que le signal détecté par RT-qPCR; «---» correspond à l'absence du transgène; « ++ », signifie que le signal fortement détecté par RT-qPCR; | | |

### 4. - Tests pathologiques in vivo pour l'évaluation de la résistance des riz transgéniques à l'infection par Magnaporthe grisea.

### Matériel végétal :

Evénements T0 à un stade de développement des plantes comprenant au moins 5 feuilles développées.
Production : Serre S2

### Inoculum :

Suspension de *Magnaporthe grisea* (10⁴ spores/mL)

### Evaluation des symptômes :

La détermination de l'intensité des symptômes se fait selon des critères biologiques en fonction de la taille et de l'allure des lésions constatées sur les feuilles. Ainsi la note 1 correspond à la visualisation de taches très légères sur la feuille, à peine perceptibles. La note 2 correspond à des taches nettes sur la feuille, mais très peu nombreuses. La note 3 correspond à des taches nettes et nombreuses sur la feuille. La note 4 correspond à des taches très nettes et très nombreuses avec présence de taches de grande taille. La note 5 correspond soit à une pourrissement total de la feuille, soit à de nombreuses taches de très grosse taille. La note 6 correspond à une plante touchée à 100 % par la maladie.

Les différences entre 1, 2, 3, 4, 5 et 6 correspondent donc à de réelles et nettes différences de symptômes. Ainsi, des plantes ayant des notes de symptômes de 6 sont mortes, les plantes 4 ou 5 sont considérées comme très sévèrement touchées. Des plantes de note 3 sont touchées mais restent acceptables. Des plantes de note 2 sont faiblement touchées et celles de note 1 ne le sont que très faiblement.

Les résultats sont présentés sous forme d'histogrammes correspondant aux moyennes des plantes par événement. L'intervalle de confiance est représenté pour chaque histogramme.

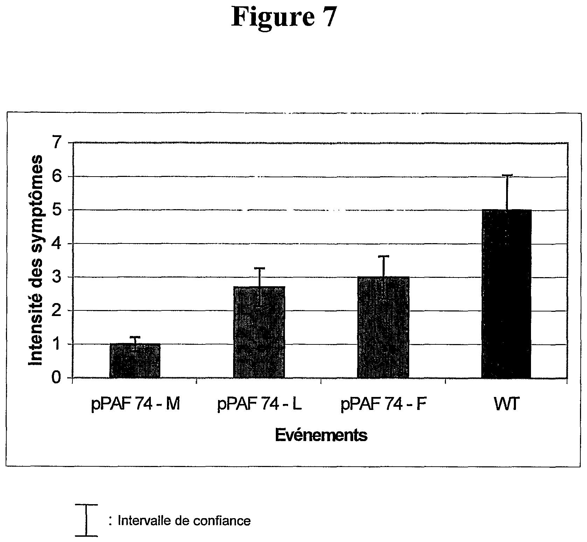

La figure 7 présente l'intensité des symptômes notée pour chaque événement.

Les résultats du test *in vivo* sont clairement corrélés avec les analyses moléculaires : la détection des longs transcrits issus de notre construit est complètement corrélée avec une intensité des symptômes plus faible et donc une résistance de la plante transgénique.

| Evénement | Présence du transgène | RT-PCRq : Detection des longs ARN | Intensité des symptômes 15 jours après infection |
|---|---|---|---|
| WT | Non | --- | 5-6 |
| M | Oui | -/+ | 1 |
| L | Oui | + | 2-3 |
| F | Oui | ++ | 2-3 |

| | | | |
|---|---|---|---|
| -/+ : détection faible + : détection ++ : détection forte --- : pas de détection | | | |

## Revendications

1. Procédé de production d'une plante résistante à un champignon phytopathogène comprenant les étapes suivantes
a) introduction dans la cellule végétale d'un construit comprenant :
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice,
b) mise en culture des cellules transformées dans des conditions permettant la transcription du construit,
c) sélection des cellules transformées,
d) régénération d'une plante à partir des cellules transformées,

2. Procédé de production d'une plante résistante à un champignon phytopathogène comprenant les étapes suivantes :
a) introduction dans une plante d'un construit comprenant :
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice,
b) mise en culture des plantes transformées dans des conditions permettant la transcription du construit
c) sélection des plantes transformées.

3. Procédé de production d'une cellule végétale résistante à un champignon phytopathogène comprenant les étapes suivantes
a) introduction dans une cellule végétale d'un construit comprenant :
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucleotides identique à la séquence complémentaire dudit fragment de gène essentiel,
- une séquence de régulation terminatrice,
b) sélection des cellules transformées,
c) mise en culture des cellules transformées dans des conditions permettant la transcription du construit.

4. Procédé de production d'une cellule végétale ou d'une plante résistante à un champignon phytopathogène selon l'une des revendications 1 à 3 **caractérisé en ce que** les séquences nucléotidiques sens et antisens sont séparées par un polynucléotide ne présentant pas d'homologie avec le gène du champignon.

5. Procédé de production d'une cellule végétale ou d'une plante résistante à un champignon phytopathogène selon l'une des revendications 1 à 3 **caractérisé en ce que** les séquences nucléotidiques sens et antisens sont de tailles différentes.

6. Procédé de production d'une cellule végétale ou d'une plante résistante à un champignon phytopathogène selon l'une des revendications 1 à 5 **caractérisé en ce que** le gène de champignon est un gène essentiel au champignon.

7. Procédé de production d'une cellule végétale ou d'une plante résistante à un champignon phytopathogène selon la revendication 6 **caractérisé en ce que** le gène de champignon est choisi dans le groupe comprenant les gènes suivants : *erg11, erg6, aur1, ipt, ef2, ef3*, *met4, met30, ilv5,* le gène codant pour la béta-tubuline.

8. Procédé de production d'une cellule végétale ou d'une plante résistante à un champignon phytopathogène selon la revendication 7 **caractérisé en ce que** le gène de champignon est le gène codant pour la béta-tubuline.

9. Procédé de production d'une cellule végétale ou d'une plante résistante à un champignon phytopathogène selon la revendication 8 **caractérisé en ce que** la séquence d'ADN est représentée par l'identifiant de séquence SEQ ID NO 4.

10. Procédé de production selon la revendication 9 **caractérisé en ce que** la plante est le tabac et champignon phytopathogène *Cercospora nicotianae.*

11. Procédé de production d'une cellule végétale ou d'une plante résistante à un champignon phytopathogène selon l'une des revendications 1 à 5 **caractérisé en ce que** le gène de champignon est un gène essentiel à la pathogénicité du champignon.

12. Procédé de production d'une cellule végétale ou d'une plante résistante à un champignon phytopathogène selon la revendication 11 **caractérisé en ce que** ce que le gène de champignon est choisi dans le groupe comprenant les gènes suivants *tri5, fum5,* 763, le gène codant pour la polygalacturonase, *buf.*

13. Procédé de production d'une cellule végétale ou d'une plante résistante à un champignon phytopathogène selon la revendication 12 **caractérisé en ce que** ce que le gène de champignon est le gène *buf.*

14. Procédé de production d'une cellule végétale ou d'une plante résistante à un champignon phytopathogène selon la revendication 13 **caractérisée en ce que** la séquence d'ADN est représentée par l'identifiant de séquence SEQ ID NO 13.

15. Procédé de production selon la revendication 14 **caractérisé en ce que** la plante est le riz et le champignon phtyopathogène *Magnaporthe grisea.*

16. Plante résistante à un champignon phytopathogène comprenant un construit **caractérisé en ce qu'**il comprend
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice.

17. Cellule végétale résistante à un champignon phytopathogène comprenant un construit **caractérisé en ce qu'**elle comprend
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice.

18. Cellule végétale ou plante résistante à un champignon phytopathogène selon l'une des revendications 16 ou 17 dans laquelle les séquences nucléotidiques sens et antisens sont séparées par un polynucléotide ne présentant pas d'homologie avec le gène cible.

19. Cellule végétale ou plante résistante à un champignon phytopathogène selon l'une des revendications 16 ou 17 dans laquelle les séquences nucléotidiques sens et antisens sont de taille différente.

20. Cellule végétale ou plante résistante à un champignon phytopathogène selon l'une des revendications 16 à 19 **caractérisée en ce que** le gène de champignon est un gène essentiel au champignon

21. Cellule végétale ou plante résistante à un champignon phytopathogène selon la revendication 20 **caractérisée en ce que** le gène de champignon est choisi dans le groupe comprenant les gènes suivants : *erg11, erg6, aur1, ipt, ef3, ef2, met4, met30, ilv5,* le gène codant pour la béta-tubuline.

22. Cellule végétale ou plante résistante à un champignon phytopathogène selon la revendication 21 **caractérisée en ce que** le gène de champignon est le gène codant pour la béta-tubuline.

23. Cellule végétale ou plante résistante à un champignon phytopathogène selon la revendication 22 **caractérisée en ce que** la séquence d'ADN est représentée par l'identifiant de séquence SEQ ID NO 4.

24. Cellule végétale ou plante résistante à un champignon phytopathogène selon l'une des revendications 16 à 19 **caractérisée en ce que** le gène de champignon est un gène essentiel à la pathogénicité du champignon.

25. Cellule végétale ou plante résistante à un champignon phytopathogène selon la revendication 24 **caractérisée en ce que** ce que le gène de champignon est choisi dans le groupe comprenant les gènes suivants *tri5, fum5, 763,* le gène codant pour la polygalacturonase, *buf.*

26. Cellule végétale ou plante résistante à un champignon phytopathogène selon la revendication 25 **caractérisée en ce que** ce que le gène de champignon est le gène *buf.*

27. Cellule végétale ou plante résistante à un champignon phytopathogène selon la revendication 26 **caractérisée en ce que** la séquence d'ADN est représentée par l'identifiant de séquence SEQ ID NO 13.

28. Plante résistante à un champignon phytopathogène **caractérisée en ce qu'**elle contient des cellules selon l'une des revendications 17 à 27.

29. Plante résistante à un champignon phytopathogène selon l'une des revendications 16, 18 à 28 **caractérisée en ce qu'**elle est choisie parmi les monocotylédones et notamment le blé, le maïs, le riz.

30. Riz résistant à *Magnaporthe grisea* selon la revendication 29, la séquence d'ADN étant représentée par l'identifiant de séquence SEQ ID NO 13.

31. Plante résistante à un champignon phytopathogène selon l'une des revendications 16, 18 à 28 **caractérisée en ce qu'**elle est choisie parmi les dicotylédones et notamment le colza, le soja, le tabac, le coton.

32. Tabac résistant à *Cercospora nicotianae* selon la revendication 31, la séquence d'ADN étant représentée par l'identifiant de séquence SEQ ID NO 4.

33. Procédé pour l'identification d'un gène essentiel au développement ou à la pathogénicité d'un champignon phytopathogène comprenant les étapes suivantes :
a) transformation d'une cellule végétale ou d'une plante avec un construit comprenant
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice
b) mise en contact des cellules ou des plantes ainsi transformées avec le champignon phytopathogène
c) étude du phénotype résultant,
d) caractérisation du gène correspondant à la séquence de nucléotides ainsi insérée.

34. Procédé pour inhiber l'expression d'un gène de champignon comprenant les étapes suivantes :
a) transformation d'une cellule végétale avec un construit comprenant
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucleotides identique à la séquence complémentaire dudit fragment de gène essentiel,
- une séquence de régulation terminatrice.
b) Sélection des cellules transformées
c) mise en culture des cellules ainsi transformées dans des conditions permettant la transcription dudit construit
d) mise en contact des cellules avec le champignon.

35. Procédé pour inhiber l'expression d'un gène de champignon selon la revendication 34 comprenant une étape supplémentaire de régénération des cellules transformées.

36. Procédé pour inhiber l'expression d'un gène de champignon comprenant les étapes suivantes:
a) transformation d'une plante avec un construit comprenant
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice.
b) Sélection des cellules transformées
c) mise en culture des plantes ainsi transformées dans des conditions permettant la transcription dudit construit
d) mise en contact des plantes avec le champignon.

37. Procédé pour réduire l'expression d'un gène de champignon comprenant les étapes suivantes :
a) transformation d'une cellule végétale avec un construit comprenant
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice.
b) Sélection des cellules transformées,
c) mise en culture des cellules ainsi transformées dans des conditions permettant la transcription dudit construit
d) mise en contact des cellules avec le champignon.

38. Procédé pour réduire l'expression d'un gène de champignon selon la revendication 37 comprenant une étape supplémentaire de régénération des cellules transformées.

39. Procédé pour réduire l'expression d'un gène de champignon comprenant les étapes suivantes :
a) transformation d'une plante avec un construit comprenant
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice.
b) Sélection des plantes transformées
c) mise en culture des plantes ainsi transformées dans des conditions permettant la transcription dudit construit
d) mise en contact des plantes avec le champignon.

40. Utilisation d'un construit pour créer une cellule végétale ou une plante résistante à un champignon, ledit construit comprenant :
- une séquence de régulation promotrice fonctionnelle dans les cellules végétales,
- une séquence d'ADN qui lorsqu'elle est transcrite génère une molécule d'ARN comprenant au moins une séquence sens et une séquence antisens, ladite séquence sens comprend une séquence d'au moins 19 nucléotides identique à un fragment d'un gène essentiel à la vie du champignon ou à sa phytopathogénicité, ladite séquence antisens comprend une séquence d'au moins 19 nucléotides identique à la séquence complémentaire dudit fragment de gène essentiel,
- une séquence de régulation terminatrice.

## Patentansprüche

1. Verfahren zur Herstellung einer Planze, die gegen einen phytopathogenen Pilz resistent ist, umfassend die folgenden Schritte:
a) Einführen eines Konstrukts, das Folgendes umfasst, in die Pflanzenzelle:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz,
b) Kultivieren der transformierten Zellen unter Bedingungen, die die Transkription des Konstrukts gestatten,
c) Selektieren der transformierten Zellen,
d) Regenerieren einer Pflanze aus den transformierten Zellen.

2. Verfahren zur Herstellung einer Planze, die gegen einen phytopathogenen Pilz resistent ist, umfassend die folgenden Schritte:
a) Einführen eines Konstrukts, das Folgendes umfasst, in eine Pflanze:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz,
b) Kultivieren der transformierten Zellen unter Bedingungen, die die Transkription des Konstrukts gestatten,
c) Selektieren der transformierten Pflanzen.

3. Verfahren zur Herstellung einer Planzenzelle, die gegen einen phytopathogenen Pilz resistent ist, umfassend die folgenden Schritte:
a) Einführen eines Konstrukts, das Folgendes umfasst, in eine Pflanzenzelle:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz,
b) Selektieren der transformierten Zellen,
c) Kultivieren der transformierten Zellen unter Bedingungen, die die Transkription des Konstrukts gestatten.

4. Verfahren zur Herstellung einer Pflanzenzelle oder einer Pflanze, die gegen einen phytopathogenen Pilz resistent ist, nach einem der Ansprüche 1 bis 3, **dadurch gekennzeichnet, dass** die Sense- und Antisense-Nukleotidsequenzen durch ein Polynukleotid, das keine Homologie mit dem Gen des Pilzes aufweist, getrennt sind.

5. Verfahren zur Herstellung einer Pflanzenzelle oder einer Pflanze, die gegen einen phytopathogenen Pilz resistent ist, nach einem der Ansprüche 1 bis 3, **dadurch gekennzeichnet, dass** die Sense- und Antisense-Nukleotidsequenzen eine unterschiedliche Größe aufweisen.

6. Verfahren zur Herstellung einer Pflanzenzelle oder einer Pflanze, die gegen einen phytopathogenen Pilz resistent ist, nach einem der Ansprüche 1 bis 5, **dadurch gekennzeichnet, dass** es sich bei dem Pilzgen um ein Gen, das für den Pilz essentiell ist, handelt.

7. Verfahren zur Herstellung einer Pflanzenzelle oder einer Pflanze, die gegen einen phytopathogenen Pilz resistent ist, nach Anspruch 6, **dadurch gekennzeichnet, dass** das Pilzgen aus der Gruppe umfassend die folgenden Gene ausgewählt ist: *erg11, erg6, aur1, ipt, ef2, ef3, met4, met30, ilv5,* das beta-Tubulin-Codiergen.

8. Verfahren zur Herstellung einer Pflanzenzelle oder einer Pflanze, die gegen einen phytopathogenen Pilz resistent ist, nach Anspruch 7, **dadurch gekennzeichnet, dass** es sich bei dem Pilzgen um das beta-Tubulin-Codiergen handelt.

9. Verfahren zur Herstellung einer Pflanzenzelle oder einer Pflanze, die gegen einen phytopathogenen Pilz resistent ist, nach Anspruch 8, **dadurch gekennzeichnet, dass** die DNA-Sequenz der Sequenzkennzeichnung SEQ ID NO: 4 entspricht.

10. Verfahren zur Herstellung nach Anspruch 9, **dadurch gekennzeichnet, dass** es sich bei der Pflanze um den Tabak und bei dem phytopathogenen Pilz um *Cercospora nicotianae* handelt.

11. Verfahren zur Herstellung einer Pflanzenzelle oder einer Pflanze, die gegen einen phytopathogenen Pilz resistent ist, nach einem der Ansprüche 1 bis 5, **dadurch gekennzeichnet, dass** es sich bei dem Pilzgen um ein Gen, das für die Pathogenität des Pilzes essentiell ist, handelt.

12. Verfahren zur Herstellung einer Pflanzenzelle oder einer Pflanze, die gegen einen phytopathogenen Pilz resistent ist, nach Anspruch 11, **dadurch gekennzeichnet, dass** das Pilzgen aus der Gruppe umfassend die folgenden Gene ausgewählt ist: *tri5, fum5,* 763, das Polygalacturonase-Codiergen, *buf.*

13. Verfahren zur Herstellung einer Pflanzenzelle oder einer Pflanze, die gegen einen phytopathogenen Pilz resistent ist, nach Anspruch 12, **dadurch gekennzeichnet, dass** es sich bei dem Pilzgen um das *buf*-Gen handelt.

14. Verfahren zur Herstellung einer Pflanzenzelle oder einer Pflanze, die gegen einen phytopathogenen Pilz resistent ist, nach Anspruch 13, **dadurch gekennzeichnet, dass** die DNA-Sequenz der Sequenzkennzeichnung SEQ ID NO: 13 entspricht.

15. Verfahren zur Herstellung nach Anspruch 14, **dadurch gekennzeichnet, dass** es sich bei der Pflanze um den Reis und bei dem phytopathogenen Pilz um *Magnaporthe grisea* handelt.

16. Pflanze, die gegen einen phytopathogenen Pilz resistent ist, umfassend ein Konstrukt, **dadurch gekennzeichnet, dass** es Folgendes umfasst:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz.

17. Pflanzenzelle, die gegen einen phytopathogenen Pilz resistent ist, umfassend ein Konstrukt, **dadurch gekennzeichnet, dass** es Folgendes umfasst:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz.

18. Pflanzenzelle oder Pflanze, die gegen einen phytopathogenen Pilz resistent ist, nach einem der Ansprüche 16 oder 17, worin die Sense- und Antisense-Nukleotidsequenzen durch ein Polynukleotid, das keine Homologie mit dem Zielgen aufweist, getrennt sind.

19. Pflanzenzelle oder Pflanze, die gegen einen phytopathogenen Pilz resistent ist, nach einem der Ansprüche 16 oder 17, worin die Sense- und Antisense-Nukleotidsequenzen eine unterschiedliche Größe aufweisen.

20. Pflanzenzelle oder Pflanze, die gegen einen phytopathogenen Pilz resistent ist, nach einem der Ansprüche 16 bis 19, **dadurch gekennzeichnet, dass** es sich bei dem Pilzgen um ein Gen, das für den Pilz essentiell ist, handelt.

21. Pflanzenzelle oder Pflanze, die gegen einen phytopathogenen Pilz resistent ist, nach Anspruch 20, **dadurch gekennzeichnet, dass** das Pilzgen aus der Gruppe umfassend die folgenden Gene ausgewählt ist: *erg11, erg6, aur1, ipt, ef3, ef2, met4, met30, ilv5,* das beta-Tubulin-Codiergen.

22. Pflanzenzelle oder Pflanze, die gegen einen phytopathogenen Pilz resistent ist, nach Anspruch 21, **dadurch gekennzeichnet, dass** es sich bei dem Pilzgen um das beta-Tubulin-Codiergen handelt.

23. Pflanzenzelle oder Pflanze, die gegen einen phytopathogenen Pilz resistent ist, nach Anspruch 22, **dadurch gekennzeichnet, dass** die DNA-Sequenz der Sequenzkennzeichnung SEQ ID NO: 4 entspricht.

24. Pflanzenzelle oder Pflanze, die gegen einen phytopathogenen Pilz resistent ist, nach einem der Ansprüche 16 bis 19, **dadurch gekennzeichnet, dass** es sich bei dem Pilzgen um ein Gen, das für die Pathogenität des Pilzes essentiell ist, handelt.

25. Pflanzenzelle oder Pflanze, die gegen einen phytopathogenen Pilz resistent ist, nach Anspruch 24, **dadurch gekennzeichnet, dass** das Pilzgen aus der Gruppe umfassend die folgenden Gene ausgewählt ist: *tri5*, *fum5,* 763, das Polygalacturonase-Codiergen, *buf.*

26. Pflanzenzelle oder Pflanze, die gegen einen phytopatogenen Pilz resistent ist, nach Anspruch 25, **dadurch gekennzeichnet, dass** es sich bei dem Pilzgen um das buf-Gen handelt.

27. Pflanzenzelle oder Pflanze, die gegen einen phytopathogenen Pilz resistent ist, nach Anspruch 26, **dadurch gekennzeichnet, dass** die DNA-Sequenz der Sequenzkennzeichnung SEQ ID NO: 13 entspricht.

28. Pflanze, die gegen einen phytopathogenen Pilz resistent ist, **dadurch gekennzeichnet, dass** sie Zellen nach einem der Ansprüche 17 bis 27 enthält.

29. Pflanze, die gegen einen phytopathogenen Pilz resistent ist, nach einem der Ansprüche 16, 18 bis 28, **dadurch gekennzeichnet, dass** sie aus den Monokotyledonen und insbesondere aus Weizen, Mais, Reis ausgewählt ist.

30. Reis, der gegen *Magnaporthe grisea* resistent ist, nach Anspruch 29, wobei die DNA-Sequenz der Sequenzkennzeichnung SEQ ID NO: 13 entspricht.

31. Pflanze, die gegen einen phytopathogenen Pilz resistent ist, nach einem der Ansprüche 16, 18 bis 28, **dadurch gekennzeichnet, dass** sie aus den Dikotyledonen und insbesondere aus Raps, Soja, Tabak, Baumwolle ausgewählt ist.

32. Tabak, der gegen *Cercospora nicotianae* resistent ist, nach Anspruch 31, wobei die DNA-Sequenz der Sequenzkennzeichnung SEQ ID NO: 4 entspricht.

33. Verfahren zum Identifizieren eines Gens, das für die Entwicklung oder die Pathogenität eines phytopathogenen Pilzes essentiell ist, umfassend die folgenden Schritte:
a) Transformieren einer Pflanzenzelle oder einer Pflanze mit einem Konstrukt, das Folgendes umfasst:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz,
b) Inkontaktbringen der so transformierten Zellen oder Pflanzen mit dem phytopathogenen Pilz,
c) Untersuchen des entstandenen Phänotyps,
d) Charakterisieren des Gens, das der so insertierten Nukleotidsequenz entspricht.

34. Verfahren zum Hemmen der Expression eines Pilzgens, umfassend die folgenden Schritte:
a) Transformieren einer Pflanzenzelle mit einem Konstrukt, das Folgendes umfasst:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz,
b) Selektieren der transformierten Zellen,
c) Kultivieren der so transformierten Zellen unter Bedingungen, die die Transkription des Konstrukts gestatten,
d) Inkontaktbringen der Zellen mit dem Pilz.

35. Verfahren zum Hemmen der Expression eines Pilzgens nach Anspruch 34, das einen zusätzlichen Schritt der Regeneration der transformierten Zellen umfasst.

36. Verfahren zum Hemmen der Expression eines Pilzgens, umfassend die folgenden Schritte:
a) Transformieren einer Pflanze mit einem Konstrukt, das Folgendes umfasst:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz,
b) Selektieren der transformierten Zellen,
c) Kultivieren der so transformierten Pflanzen unter Bedingungen, die die Transkription des Konstrukts gestatten,
d) Inkontaktbringen der Pflanzen mit dem Pilz.

37. Verfahren zum Vermindern der Expression eines Pilzgens, umfassend die folgenden Schritte:
a) Transformieren einer Pflanzenzelle mit einem Konstrukt, das Folgendes umfasst:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz,
b) Selektieren der transformierten Zellen,
c) Kultivieren der so transformierten Zellen unter Bedingungen, die die Transkription des Konstrukts gestatten,
d) Inkontaktbringen der Zellen mit dem Pilz.

38. Verfahren zum Vermindern der Expression eines Pilzgens nach Anspruch 37, umfassend einen zusätzlichen Schritt der Regeneration der transformierten Zellen.

39. Verfahren zum Vermindern der Expression eines Pilzgens, umfassend die folgenden Schritte:
a) Transformieren einer Pflanze mit einem Konstrukt, das Folgendes umfasst:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz,
b) Selektieren der transformierten Pflanzen,
c) Kultivieren der so transformierten Pflanzen unter Bedingungen, die die Transkription des Konstrukts gestatten,
d) Inkontaktbringen der Pflanzen mit dem Pilz.

40. Verwendung eines Konstrukts zur Erzeugung einer Pflanzenzelle oder einer Pflanze, die gegen einen Pilz resistent ist, wobei das Konstrukt Folgendes umfasst:
- eine Promoterregulationssequenz, die in Pflanzenzellen funktionell ist,
- eine DNA-Sequenz, die, wenn sie transkribiert wird, ein RNA-Molekül erzeugt, das mindestens eine Sense-Sequenz und eine Antisense-Sequenz umfasst, wobei die Sense-Sequenz eine Sequenz von mindestens 19 Nukleotiden umfasst, die mit einem Fragment eines Gens, das für das Leben des Pilzes oder seine Phytopathogenität essentiell ist, identisch ist, wobei die Antisense-Sequenz eine Sequenz von mindestens 19 Nukleotiden umfasst, die mit der Sequenz, die zu dem Fragment des essentiellen Gens komplementär ist, identisch ist,
- eine Terminationsregulationssequenz.

## Claims

1. Method of producing a plant resistant to a phytopathogenic fungus, comprising the following steps:
a) introducing into a plant cell a construct comprising:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence,
b) placing the transformed cells in culture under conditions that allow the transcription of the construct,
c) selecting the transformed cells,
d) regenerating a plant from the transformed cells.

2. Method of producing a plant resistant to a phytopathogenic fungus, comprising the following steps:
a) introducing into a plant a construct comprising:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence,
b) placing the transformed plants in culture under conditions that allow the transcription of the construct,
c) selecting the transformed plants.

3. Method of producing a plant cell resistant to a phytopathogenic fungus, comprising the following steps:
a) introducing into a plant cell a construct comprising:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence,
b) selecting the transformed cells,
c) placing the transformed cells in culture under conditions that allow the transcription of the construct.

4. Method of producing a plant cell or a plant resistant to a phytopathogenic fungus according to one of Claims 1 to 3, **characterized in that** the sense and antisense nucleotide sequences are separated by a polynucleotide that does not exhibit any homology with the fungal gene.

5. Method of producing a plant cell or a plant resistant to a phytopathogenic fungus according to one of Claims 1 to 3, **characterized in that** the sense and antisense nucleotide sequences have different sizes.

6. Method of producing a plant cell or a plant resistant to a phytopathogenic fungus according to one of Claims 1 to 5, **characterized in that** the fungal gene is a gene essential to the fungus.

7. Method of producing a plant cell or a plant resistant to a phytopathogenic fungus according to Claim 6, **characterized in that** the fungal gene is chosen from the group comprising the following genes: *erg11, erg6, aur1, ipt, ef2, ef3, met4, met30, ilv5,* and the gene encoding beta-tubulin.

8. Method of producing a plant cell or a plant resistant to a phytopathogenic fungus according to Claim 7, **characterized in that** the fungal gene is the gene encoding beta-tubulin.

9. Method of producing a plant cell or a plant resistant to a phytopathogenic fungus according to Claim 8, **characterized in that** the DNA sequence is represented by the sequence identifier SEQ ID No. 4.

10. Method of production according to Claim 9, **characterized in that** the plant is tobacco and the phytopathogenic fungus is *Cercospora nicotianae.*

11. Method of producing a plant cell or a plant resistant to a phytopathogenic fungus according to one of Claims 1 to 5, **characterized in that** the fungal gene is a gene essential to the pathogenicity of the fungus.

12. Method of producing a plant cell or a plant resistant to a phytopathogenic fungus according to Claim 11, **characterized in that** the fungal gene is chosen from the group comprising the following genes: *tri5, fum5, 763,* the gene encoding polygalacturonase, and *buf.*

13. Method of producing a plant cell or a plant resistant to a phytopathogenic fungus according to Claim 12, **characterized in that** the fungal gene is the *buf* gene.

14. Method of producing a plant cell or a plant resistant to a phytopathogenic fungus according to Claim 13, **characterized in that** the DNA sequence is represented by the sequence identifier SEQ ID No. 13.

15. Method of production according to Claim 14, **characterized in that** the plant is rice and the phytopathogenic fungus is *Magnaporthe grisea.*

16. Plant resistant to a phytopathogenic fungus, comprising a construct **characterized in that** it comprises:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence.

17. Plant cell resistant to a phytopathogenic fungus, comprising a construct **characterized in that** it comprises:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence.

18. Plant cell or plant resistant to a phytopathogenic fungus according to either of Claims 16 and 17, in which the sense and antisense nucleotide sequences are separated by a polynucleotide that does not exhibit any homology with the target gene.

19. Plant cell or plant resistant to a phytopathogenic fungus according to either of Claims 16 and 17, in which the sense and antisense nucleotide sequences have different sizes.

20. Plant cell or plant resistant to a phytopathogenic fungus according to one of Claims 16 to 19, **characterized in that** the fungal gene is a gene essential to the fungus.

21. Plant cell or plant resistant to a phytopathogenic fungus according to Claim 20, **characterized in that** the fungal gene is chosen from the group comprising the following genes: *erg11, erg6, aur1, ipt, ef3*, *ef2,* met4, *met30, ilv5,* and the gene encoding beta-tubulin.

22. Plant cell or plant resistant to a phytopathogenic fungus according to Claim 21, **characterized in that** the fungal gene is the gene encoding beta-tubulin.

23. Plant cell or plant resistant to a phytopathogenic fungus according to Claim 22, **characterized in that** the DNA sequence is represented by the sequence identifier SEQ ID No. 4.

24. Plant cell or plant resistant to a phytopathogenic fungus according to one of Claims 16 to 19, **characterized in that** the fungal gene is a gene essential to the pathogenicity of the fungus.

25. Plant cell or plant resistant to a phytopathogenic fungus according to Claim 24, **characterized in that** the fungal gene is chosen from the group comprising the following genes: *tri5, fum5, 763,* the gene encoding polygalacturonase, and *buf.*

26. Plant cell or plant resistant to a phytopathogenic fungus according to Claim 25, **characterized in that** the fungal gene is the *buf* gene.

27. Plant cell or plant resistant to a phytopathogenic fungus according to Claim 26, **characterized in that** the DNA sequence is represented by the sequence identifier SEQ ID No. 13.

28. Plant resistant to a phytopathogenic fungus, **characterized in that** it contains cells according to one of Claims 17 to 27.

29. Plant resistant to a phytopathogenic fungus according to one of Claims 16, and 18 to 28, **characterized in that** it is chosen from monocotyledons, and in particular wheat, maize and rice.

30. Rice resistant to *Magnaporthe grisea* according to Claim 29, the DNA sequence being represented by the sequence identifier SEQ ID No. 13.

31. Plant resistant to a phytopathogenic fungus according to one of Claims 16, and 18 to 28, **characterized in that** it is chosen from dicotyledons, and in particular rapeseed, soybean, tobacco and cotton.

32. Tobacco resistant to *Cercospora nicotianae* according to Claim 31, the DNA sequence being represented by the sequence identifier SEQ ID No. 4.

33. Method for identifying a gene essential to the development or to the pathogenicity of a phytopathogenic fungus, comprising the following steps:
a) transforming a plant cell or a plant with a construct comprising:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence,
b) bringing the cells or the plants thus transformed into contact with the phytopathogenic fungus,
c) studying the resulting phenotype,
d) characterizing the gene corresponding to the sequence of nucleotides thus inserted.

34. Method for inhibiting the expression of a fungal gene, comprising the following steps:
a) transforming a plant cell with a construct comprising:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence,
b) selecting the transformed cells,
c) placing the cells thus transformed in culture under conditions that allow the transcription of said construct,
d) bringing the cells into contact with the fungus.

35. Method for inhibiting the expression of a fungal gene according to Claim 34, comprising an additional step of regenerating the transformed cells.

36. Method for inhibiting the expression of a fungal gene, comprising the following steps:
a) transforming a plant with a construct comprising:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence,
b) selecting the transformed cells,
c) placing the plants thus transformed in culture under conditions that allow the transcription of said construct,
d) bringing the plants into contact with the fungus.

37. Method for reducing the expression of a fungal gene, comprising the following steps:
a) transforming a plant cell with a construct comprising:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence,
b) selecting the transformed cells,
c) placing the cells thus transformed in culture under conditions that allow the transcription of said construct,
d) bringing the cells into contact with the fungus.

38. Method for reducing the expression of a fungal gene according to Claim 37, comprising an additional step of regenerating the transformed cells.

39. Method for reducing the expression of a fungal gene, comprising the following steps:
a) transforming a plant with a construct comprising:
a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence,
b) selecting the transformed cells,
c) placing the plants thus transformed in culture under conditions that allow the transcription of said construct,
d) bringing the plants into contact with the fungus.

40. Use of a construct for creating a plant cell or a plant resistant to a fungus, said construct comprising:
- a promoter regulatory sequence that is functional in plant cells,
- a DNA sequence which, when it is transcribed, generates an RNA molecule comprising at least one sense sequence and one antisense sequence, said sense sequence comprises a sequence of at least 19 nucleotides which is identical to a fragment of a gene essential to the life of the fungus or to its phytopathogenicity, said antisense sequence comprises a sequence of at least 19 nucleotides which is identical to the sequence complementary to said fragment of essential gene,
- a terminator regulatory sequence.
